# DEMANDE DE BREVET EUROPEEN

(11) **EP 1 845 467 A1**
(43) Date de publication de la demande: **17.10.2007**
(21) Numéro de dépôt: 07290435.2
(22) Date de dépôt: 10.04.2007
(51) Int. Cl.: G06F 19/00

(54) **Installation de recueil de données biomédicales et mesure de constantes biologiques par des appareils de mesure individuels**

(30) Priorité: 10.04.2006 FR 0603171
(71) Demandeur: Chemla, Alain, HK (CN); Sillam, Mickaël, 75008 Paris (FR); Guchet, Anthony, Hong Kong (HK)
(72) Inventeur: Chemla, Alain, HK (CN); Sillam, Mickaël, 75008 Paris (FR); Guchet, Anthony, Hong Kong (HK)
(74) Mandataire: Blot, Philippe Robert Emile

(57) **Abrégé**

L'installation (10) de recueil de données biomédicales comporte :
- au moins un appareil (12) de mesure d'au moins un paramètre biomédical ;
- au moins une entité centrale (40) de traitement d'informations comportant des moyens (46, 48) de stockage et de traitement du ou de chaque paramètre biomédical fourni par le ou chaque appareil (12),

Le ou chaque appareil (12) comporte une carte amovible (18) de stockage du ou de chaque paramètre biomédical mesuré par l'appareil (12) et l'installation comporte au moins une borne (22A, 22B) de collecte du ou de chaque paramètre biomédical, la ou chaque borne (22A, 22B) comportant :
- un lecteur (24) de carte propre à lire la carte amovible (18) du ou de chaque appareil (12) après que celle-ci a été extraite de l'appareil avec les paramètres stockés ; et
- des moyens (42) de transmission du ou de chaque paramètre de la carte vers l'entité centrale de traitement d'informations (40).

## Description

La présente invention concerne une installation de recueil de données biomédicales, du type comportant au moins un appareil de mesure d'au moins un paramètre biomédical et au moins une entité centrale de traitement d'informations comportant des moyens de stockage et de traitement du ou de chaque paramètre biomédical fourni par le ou chaque appareil.

De nos jours, il est nécessaire de procéder à des études épidémiologiques à grande échelle afin de connaître l'évolution sanitaire d'une population vis-à-vis de certains facteurs favorisant la maladie, ou pour connaître les réactions à la prise de certains médicaments.

Pour ce faire, il est connu de procéder à des recueils de données biomédicales.

A cet effet, un échantillon représentatif de patients est équipé d'un appareil individuel de mesure. Cet appareil permet au patient de procéder lui-même à la mesure de certains paramètres biomédicaux, tels que sa tension artérielle, son taux de cholestérol, sa glycémie, son rythme cardiaque ou tout autre paramètre.

A l'issue de l'étude ou à des stades successifs de l'étude, les paramètres mémorisés dans l'appareil de chaque participant sont recueillis, par exemple par lecture de la mémoire contenue dans l'appareil à partir d'un ordinateur portable. Les données sont ensuite transférées de l'ordinateur portable dans une unité centrale de traitement d'informations assurant le stockage des données recueillies et leur traitement afin de permettre une étude scientifique des données.

Compte tenu des importantes contraintes logistiques pour ce type d'étude, celles-ci sont limitées à un nombre réduit de patients et ne peuvent être que de durée limitée. En outre, les patients sont sélectionnés.

L'invention a pour but de proposer une installation permettant de faciliter la mise en oeuvre de telles études afin qu'elles puissent s'étendre à un très grand nombre de participants, sur une longue durée.

A cet effet, l'invention a pour objet une installation du type précité, caractérisée en ce le ou chaque appareil comporte une carte amovible de stockage du ou de chaque paramètre biomédical mesuré par l'appareil et l'installation comporte au moins une borne de collecte du ou de chaque paramètre biomédical, la ou chaque borne comportant :
- un lecteur de carte propre à lire la carte amovible du ou de chaque appareil après que celle-ci a été extraite de l'appareil avec les paramètres stockés ; et
- des moyens de transmission du ou de chaque paramètre de la carte vers l'entité centrale de traitement d'informations.

Suivant des modes particuliers de réalisation, l'installation comporte l'une ou plusieurs des caractéristiques suivantes :
- ledit appareil de mesure comporte des moyens de sauvegarde dans la carte amovible de la date et l'heure de mesure de chaque paramètre biomédical sauvegardé et la ou chaque borne comporte des moyens de lecture de la date et l'heure de mesure de chaque paramètre dans la carte ;
- la ou chaque borne de collecte comporte des moyens d'impression d'au moins un paramètre biomédical ;
- la ou chaque borne de collecte comporte des moyens de saisie d'un code et que la ou chaque carte comporte un numéro d'identification propre et un code confidentiel, la borne étant propre à lire le ou chaque paramètre biomédical stocké sur la carte seulement en cas de concordance des codes ;
- l'entité centrale de traitement d'informations comporte des premiers moyens de stockage pour le stockage du ou de chaque paramètre biomédical transmis ;
- elle comporte des seconds moyens de stockage pour la sauvegarde de paramètres de récurrence représentatifs du nombre de mesures mémorisées sur chaque carte de stockage et transmises à l'entité centrale de traitement d'informations depuis au moins une borne de collecte ; et
- l'entité centrale de traitement d'informations comporte des moyens de production de gratification en fonction des paramètres biomédicaux reçus.

L'invention sera mieux comprise à la lecture de la description qui va suivre, donnée uniquement à titre d'exemple et faite en se référant aux dessins, sur lesquels :
- la Figure 1 est une vue schématique de l'installation selon l'invention ; et
- la Figure 2 est un organigramme illustrant les flux de données.

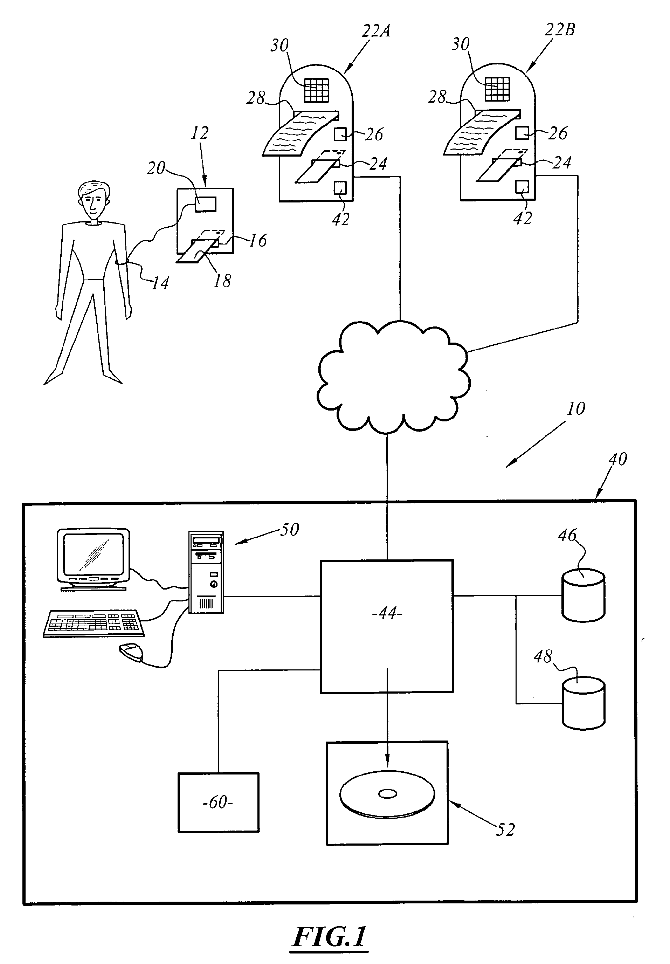

L'installation 10 illustrée sur la Figure 1 est propre à assurer le suivi sur une population très importante d'un ou de plusieurs paramètres biomédicaux. Par exemple, le paramètre suivi est la tension artérielle.

L'installation comporte pour chaque participant un appareil autonome 12 de mesure du paramètre devant être mesuré. Dans l'exemple considéré, l'appareil est un tensiomètre électronique permettant au participant de procéder lui-même à la mesure de sa propre tension artérielle.

L'appareil comporte un signe comme connu en soi, un brassard gonflable 14 ou un système de mesure de la tension artérielle au poignet auquel sont intégrés des moyens de détection du pouls et des moyens propres à effectuer une mesure de la tension artérielle.

En outre, l'appareil comporte un lecteur 16 de carte à puce ainsi qu'une carte à puce 18 amovible. Chaque carte comporte un numéro d'identification qui lui est propre et éventuellement un code confidentiel que définira l'utilisateur. L'appareil comporte des moyens 20 de calcul de la valeur de la tension artérielle et de gestion du stockage d'informations sur la carte à puce. Ces informations sont de préférence la date et l'heure de la mesure et la valeur du ou des paramètres mesurés.

Par ailleurs, l'installation comporte un ensemble de bornes 22A, 22B de collecte, chacune équipée d'un lecteur 24 pour une carte amovible 18 issue d'un appareil de mesure 12, d'un écran pour afficher différents messages.

Les bornes de collecte 22A, 22B sont avantageusement installées dans des lieux de passage des participants, tels que des pharmacies, hôpitaux, cliniques et cabinets médicaux.

Chaque borne comporte outre le lecteur de carte 24, des moyens 26 de traitement d'information propres à collecter les données copiées sur la carte et à interpréter celle-ci. La borne comporte également éventuellement des moyens 28 d'impression propres à imprimer les valeurs des paramètres mesurées ainsi que leurs dates afin de fournir ces données sous forme écrite au participant lors de chaque introduction de la carte.

En outre, avantageusement, la borne comporte éventuellement un clavier 30 permettant la saisie d'un code confidentiel afin de n'autoriser la lecture de la carte 18 qu'après que le code confidentiel propre à la carte a été saisi, ceci dans le cas où la carte comporte un code confidentiel. Dans le cas contraire, la lecture de la carte se fait directement après insertion de la carte dans la borne et le suivi de la procédure indiquée sur l'écran.

L'installation comporte en outre une entité centrale 40 de traitement d'informations. Chaque borne comporte des moyens de communication 42 avec cette entité centrale 40. A cet effet, chaque borne 22A, 22B est reliée à l'entité centrale 40 par un réseau de traitement d'informations tel que le réseau Internet. L'entité centrale 40 comporte une unité de traitement d'informations 44 associée à des premiers moyens de stockage 46 pour le stockage de paramètres biomédicaux mesurés et une seconde entité de stockage 48 pour la sauvegarde de paramètres de récurrence.

Enfin, l'entité centrale comporte des moyens 50 de saisie d'informations propres à renseigner les bases de données sauvegardées.

Par ailleurs, l'entité centrale 40 comporte des moyens de mise à disposition d'informations tels qu'un gaveur de DVD 52.

Dans l'entité centrale 40, l'unité centrale 44 de traitement comporte des moyens de calcul du nombre d'informations et de la pertinence des informations recueillies pour chaque participant. L'entité 60 comporte des moyens 60 propres à provoquer le déclenchement de l'envoi d'une gratification auprès des participants dont les informations recueillies satisfont à des critères quantitatifs et qualitatifs prédéterminés. Les moyens 60 sont constitués par exemple d'une imprimante propre à établir un chèque de remboursement de l'appareil de mesure ou de toute autre valeur.

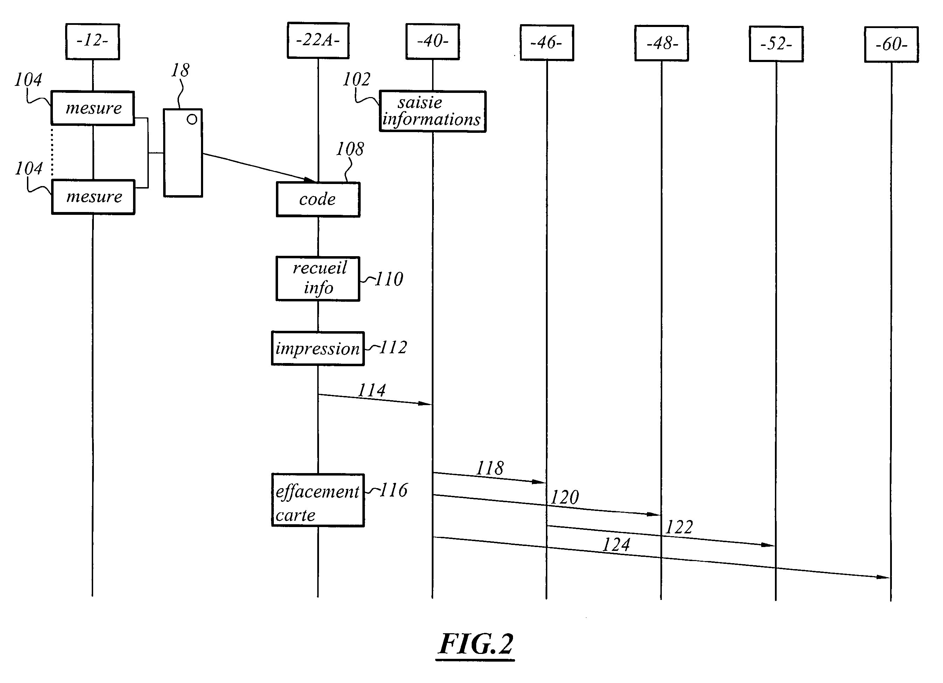

Les appareils de mesure 12, les bornes 22A, 22B et l'unité de traitement d'informations 44 mettent en oeuvre des programmes informatiques propres à gérer les flux de données, comme explicité sur l'ordinogramme de la Figure 2.

Les appareils de mesure 12 sont commercialisés à titre onéreux ou distribués gratuitement par un réseau de distribution adapté, tel que des pharmacies ou points de vente et/ou de distribution de ce type d'appareil. Lors de la remise d'un appareil de mesure à un participant à l'étude, l'appareil est équipé d'une carte à puce 18 comportant un numéro d'identification propre.

Différentes données propres au participant sont relevées par le distributeur. En particulier, ces données comportent de préférence mais non exclusivement le nom et le prénom du participant, son âge, son poids, son adresse, le nom de son médecin traitant sous contrôle de tout organisme autorisé, spécifique à chacun des pays visés par la mise en oeuvre de ce brevet. En outre, le numéro d'identification propre à la carte est relevé.

Ces différentes informations sont transmises, par exemple par courrier, à l'entité centrale 40 où un opérateur saisit en 102 l'ensemble de ces informations depuis les moyens 50. En variante, ces informations sont saisies directement par le distributeur de l'appareil et sont transmises par des moyens de télécommunication à l'entité centrale de traitement.

Le patient utilise alors, à intervalles réguliers, l'appareil de mesure. Par exemple, il procède chaque jour en 104 à une mesure du paramètre considéré, par exemple à une mesure de la tension artérielle. La date et l'heure, ainsi que chaque valeur mesurée, sont, lors de chaque utilisation, mémorisées dans la carte.

A intervalles réguliers, par exemple chaque mois, la carte 18 est introduite dans l'appareil 12 pour collecter les informations ou extraite de l'appareil si la carte est intégrée à l'appareil par le participant et celui-ci se rend auprès d'une borne de collecte par exemple 22A où il insert sa carte dans le lecteur. Il compose alors son code confidentiel en 108 si nécessaire. Après quoi, la borne procède en 110 à la lecture de la carte et au recueil des informations contenues dans celles-ci. Les informations sont alors éventuellement imprimées par la borne en 112 pour fournir une version écrite au participant des informations nouvellement mesurées depuis la dernière lecture de la carte.

Les informations recueillies sont transmises par la borne à l'entité centrale 40 en 114. Suivant un premier mode de réalisation, cette transmission s'effectue lors de chaque lecture d'une carte.

En variante, la borne 22A comporte des moyens de stockage des informations lues sur plusieurs cartes et des moyens pour transmettre les informations lues sur plusieurs cartes seulement à intervalles prédéterminés, par exemple à une fréquence régulière, par exemple chaque jour, ou encore après un nombre prédéterminé de cartes lues.

Les informations communiquées depuis la borne sont le numéro d'identification de la carte, la date des mesures, ainsi que les valeurs des paramètres mesurés.

A l'issue de la transmission, le serveur envoie un accusé de réception des informations sur la carte à puce. Après recueil des informations depuis la borne, le participant reprend sa carte et, de retour chez lui, lorsque celle-ci est réinsérée dans le dispositif 12, les données de la carte sont alors effacées de celle-ci lorsque le dispositif 12 reçoit l'accusé de réception et les nouvelles données seront alors enregistrées.

Les informations reçues par l'entité centrale 40 sont stockées dans les deux bases de données 46, 48. Les informations biomédicales sont stockées en 118 dans la première base 46 et sont associées de manière anonyme à chaque participant. Ainsi, seul le numéro d'identification de la carte, et des paramètres caractéristiques du participant tels que son âge et son poids sont mémorisés en étant associés à chacune des valeurs mesurées.

Au contraire, dans la seconde base 48, les identifiants du participant tels que son nom, son adresse et toute autre information permettant de le reconnaître sont mémorisés en 120. Dans cette même base 48 sont mémorisées des informations relatives au nombre de fois où le participant a procédé à une lecture de sa carte depuis une borne de collecte, ainsi que la fréquence de ces lectures.

Les données contenues dans la première base sont périodiquement gravées en 122 sur des CD ou DVD ou tout support et transmises à des laboratoires de recueil des données épidémiologiques en vue de l'établissement d'études destinées aux laboratoires pharmaceutiques ou à tout organisme intéressé, afin de connaître l'état sanitaire d'une population, ou encore connaître sa réaction au traitement par certains médicaments. Les données ainsi collectées pourront également renseigner sur l'observance à traitement d'une population de malades et permettre ainsi l'élaboration d'études d'observance et de pharmacovigilance.

En fonction des informations relatives au nombre de valeurs de paramètres fournis par le participant, des ordres d'impression d'une gratification sont adressés en 124 à l'imprimante 60.

On conçoit qu'une telle installation est particulièrement adaptée au traitement d'un très grand nombre de participants sur une très longue période puisque, grâce à la mise en oeuvre de cartes amovibles et grâce à la présence de bornes dans des lieux fréquentés par les participants, la collecte des informations relevées peut s'effectuer très facilement, tant pour les personnes en charge de l'étude que pour les participants.

## Revendications

1. Installation (10) de recueil de données biomédicales comportant :
- au moins un appareil (12) de mesure d'au moins un paramètre biomédical d'un participant ;
- au moins une entité centrale (40) de traitement d'informations comportant des premiers moyens (46) de stockage et de traitement du ou de chaque paramètre biomédical fourni par le ou chaque appareil (12),
**caractérisée en ce que** :
- le ou chaque appareil (12) comporte une carte amovible (18) de stockage du ou de chaque paramètre biomédical mesuré par l'appareil (12) ; et
- l'installation comporte au moins une borne (22A, 22B) de collecte du ou de chaque paramètre biomédical, la ou chaque borne (22A, 22B) comportant :
- un lecteur (24) de carte propre à lire la carte amovible (18) du ou de chaque appareil (12) après que celle-ci a été extraite de l'appareil avec les paramètres stockés ; et
- des moyens (42) de transmission du ou de chaque paramètre de la carte vers l'entité centrale de traitement d'informations (40),
et **en ce qu'**elle comporte des seconds moyens de stockage (48) pour la sauvegarde de paramètres de récurrence représentatifs du nombre de mesures mémorisées sur chaque carte de stockage et transmises à l'entité centrale de traitement d'informations depuis au moins une borne (22A, 22B) de collecte.

2. Installation (10) selon la revendication 1, **caractérisée en ce que** ledit appareil de mesure comporte des moyens de sauvegarde dans la carte amovible (18) de la date et l'heure de mesure de chaque paramètre biomédical sauvegardé et la ou chaque borne (22A, 22B) comporte des moyens de lecture de la date et l'heure de mesure de chaque paramètre dans la carte (18).

3. Installation selon la revendication 1 ou 2, **caractérisée en ce que** la ou chaque borne de collecte (22A, 22B) comporte des moyens (28) d'impression d'au moins un paramètre biomédical.

4. Installation selon l'une quelconque des revendications précédentes, **caractérisée en ce que** la ou chaque borne (22A, 22B) de collecte comporte des moyens (20) de saisie d'un code et que la ou chaque carte (18) comporte un numéro d'identification propre et un code confidentiel, la borne (22A, 22B) étant propre à lire le ou chaque paramètre biomédical stocké sur la carte seulement en cas de concordance des codes.

5. Installation selon l'une quelconque des revendications précédentes, **caractérisée en ce que** l'entité centrale de traitement d'informations (40) comporte des moyens (60) de production d'une gratification en fonction des paramètres biomédicaux reçus.

6. Installation selon la revendication 5, **caractérisée en ce que** les moyens (60) de production d'une gratification sont propres à produire cette gratification en fonction du nombre de mesures de paramètres mémorisés sur chaque carte et transmises à l'entité centrale de traitement d'informations par le participant.

7. Installation selon l'une quelconque des revendications précédentes, **caractérisée en ce que** les premiers et seconds moyens de stockage sont formés de deux bases de données.

8. Installation selon l'une quelconque des revendications précédentes, **caractérisée en ce qu'**elle comporte des moyens de stockage, dans les premiers moyens de stockage (46), du ou de chaque paramètre biomédical associé de manière anonyme à chaque participant.

9. Installation selon la revendication 8, **caractérisée en ce qu'**elle comporte des moyens de stockage, dans les premiers moyens de stockage (46), de manière associée à chaque paramètre biomédical, un numéro d'identification de la carte et des paramètres caractéristiques du participant tel que son âge, et son poids.

10. Installation selon l'une quelconque des revendications précédentes, **caractérisée en ce qu'**elle comporte des moyens de stockage, dans les seconds moyens de stockage (48), d'un identifiant du participant tel que son nom, son adresse et toute autre information permettant de le reconnaître.

11. Procédé de recueil de données biomédicales comportant :
- une étape de mesure d'au moins un paramètre biomédical ; et
- le traitement et le stockage des informations biomédicales mesu-rées depuis une entité centrale de traitement d'informations (40);
**caractérisé en ce qu'**il comporte les étapes consistant à :
- procéder au stockage sur une carte (18) du ou de chaque paramètre biomédical mesuré ;
- la lecture du ou de chaque paramètre biomédical depuis la carte de stockage ;
- l'envoi vers l'entité centrale de traitement d'informations (40) au travers d'un réseau de transmission de données pour le traitement et le stockage du ou de chaque paramètre biomédical ; et
- la sauvegarde de paramètres de récurrence représentatifs du nombre de mesures mémorisées sur chaque carte (18) et transmises à l'entité centrale de traitement d'informations depuis au moins une borne (22A, 22B) de collecte.

12. Procédé selon la revendication 11, **caractérisé en ce qu'**il comprend la production d'une gratification en fonction des paramètres biomédicaux reçus.
